# EUROPEAN PATENT SPECIFICATION

(11) **EP 3 178 390 B1**
(45) Date of publication and mention of the grant of the patent: **16.03.2022**
(21) Application number: 15306962.0
(22) Date of filing: 09.12.2015
(51) Int. Cl.: A61B 5/30

(54) **AUTONOMOUS BIOELECTRIC PHYSIOLOGICAL SIGNAL ACQUISITION DEVICE**
AUTONOME VORRICHTUNG ZUR ERFASSUNG BIOELEKTRISCHER PHYSIOLOGISCHER SIGNALE
DISPOSITIF AUTONOME D'ACQUISITION DE SIGNAUX PHYSIOLOGIQUES BIOÉLECTRIQUES

(43) Date of publication of application: 14.06.2017
(73) Proprietor: DREEM, 75009 Paris (FR)
(72) Inventor: SOULET DE BRUGIÈRE, Quentin, 33115 Pyla sur Mer (FR); MERCIER, Hugo, 91380 Chilly Mazarin (FR); MOHAMADABADI, Kaveh, 75016 Paris (FR)
(74) Representative: Plasseraud IP

(56) References cited:
- US-A- 5 662 688
- US-A1- 2013 116 577
- US-A1- 2014 221 850
- US-A1- 2014 276 184
- US-A1- 2015 065 908
- US-A1- 2015 080 699

## Description

### FIELD OF THE INVENTION

The instant invention relates to methods for bioelectric physiological signal acquisition and autonomous bioelectric physiological signal acquisition devices.

### BACKGROUND OF THE INVENTION

Bioelectric physiological signals generated by electrical currents within the neurons or the nerves of an individual can be detected by performing electrocardiogram or electroencephalogram for instance. Such measurements have led to breakthrough in the field of biological sciences and present huge potentials in many daily-life fields such as self-quantization, self-optimization or human-computer interfaces to name only a few.

Recently, bioelectric physiological signal acquisition devices have started to be developed with the aim of providing conformable and easy to use way of acquiring bioelectric physiological signal.

Documents WO 2015/017563, CN 103961093, CN 102512159, US 2015/080699 and WO 2014062738 describe examples of such devices.

These devices usually comprise a few electrodes to acquire an analog signal, filters and converters to obtain a digital signal and a microprocessor to process the signals and send wirelessly said signals to an external server. These devices are designed to be worn by the user during a certain period, for example a period of sleep in the case of sleep tracking devices. These devices may also act on the brain function of the user during this period, for example by emitting sound pattern to stimulate the brain of the user during sleep.

Since these devices may be worn over long periods of time, up to several hours, there is an incentive for the devices to be autonomous and able to process signals without having to communicate with external servers. This way, it is possible to minimize exposure of the user to electromagnetic fields.

However, the computational power and memory required to process the bioelectric signals imposes stringent demands on the power supply and the autonomy of the devices.

There is thus a need to further improve the autonomy of the bioelectric physiological signals acquisition while keeping a high quality and reliability of signals acquisition.

The instant invention has notably for object to improve this situation.

### SUMMARY OF THE INVENTION

To this aim, a first object of the invention is a method for bioelectric physiological signal acquisition in accordance with claim 1.

In some embodiments, one might also use one or more of the following features:
- steps /b/ to /g/ are reiterated continuously during a period of acquisition of the device;
- if an analog digital converter and/or an analog amplifier of an acquisition chain has been powered out, the following steps are periodically performed:
   /h/ powering said analog digital converter and/or analog amplifier of an acquisition chain,
   /i/ performing at least one iteration of steps /b/ to /e/,
   if the digital signal obtained from said at least one iteration is not considered saturated,
   /j/ setting back on said analog digital converter and/or analog amplifier for further iterations of steps /b/ to /e/ until said digital signal is considered saturated,
   if the digital signal obtained from said at least one iteration is considered saturated,
   /k/ powering out said analog digital converter and/or analog amplifier for further iterations of steps /b/ to /g/ until step /h/ is performed again;
- if the amplification factor currently applied to the digital signal by the programmable analog amplifier is not equal to the highest amplification factor in the list of predefined amplification factors, the following steps are periodically performed:
   /l/ selecting, in the list of predefined amplification factors, an amplification factor higher than the amplification factor currently applied to the digital signal by the programmable analog amplifier,
   /m/ updating the amplification factor of the programmable analog amplifier to the selected amplification factor, and
   /n/ performing at least one iteration of steps /b/ to /g/;
- to determine whether a digital signal is considered saturated,
   a portion of the digital signal is stored in a buffer memory, preferably a portion with a duration of at least one second,
   a ratio of saturated values to non-saturated values in the stored digital signal is computed,
   said ratio is compared to a predefined threshold, and
   if said ratio is over said predefined threshold, the digital signal is considered saturated;
- if the digital signal is considered saturated an amplification level change indicator is written in a memory of the device together with an associated time stamp, and preferably if the analog digital converter of the acquisition chain is powered out a channel power out indicator is further written in said memory together with an associated time stamp;
- the provided autonomous bioelectric physiological signal acquisition device comprises a plurality of electrodes and a plurality of respective acquisition chains respectively associated to said electrodes,
   said plurality of electrodes acquires a plurality of respective analog signals representative of a plurality of respective bioelectric physiological signals,
   said plurality of analog signals are amplified by a plurality of amplification factor by means of a respective plurality of programmable analog amplifier of said plurality of acquisition chains,
   said plurality of amplified analog signals are converted in a respective plurality of digital signals by means of a respective plurality of analog digital converters of said plurality of acquisition chains,
   said digital signals are independently processed to determine whether each digital signal of the plurality of digital signals is considered saturated,
   and for each digital signal of the plurality of digital signals that is considered saturated,
   an amplification factor, lower than the amplification factor currently applied to said digital signal by the programmable analog amplifier associated to said digital signal, is selected in a list of predefined amplification factors, and
   the amplification factor of the programmable analog amplifier associated to said digital signal is updated to the selected amplification factor.

Another object of the invention is an autonomous bioelectric physiological signal acquisition device in accordance with claim 8.

In some embodiments, one might also use one or more of the following features:
- the device comprises a plurality of electrodes and a plurality of respective acquisition chains respectively associated to said electrodes;
- the acquisition chains are integrated in a single monolithic chip incorporating programmable analog amplifiers and analog digital converters;
- the analog digital converter is of the 24bit delta-sigma (Δ-Σ) type with an output data rate between 250SPS and 32kSPS;

- the device comprises at least one battery powering at least the acquisition chain, the microcontroller and the memory;
- the device further comprises a microprocessor able to receive the digital signal from the microcontroller and to process said digital signal.

With these features, among other advantages, the autonomy of the bioelectric physiological signals acquisition is improved and a high quality and reliability of signals acquisition is provided.

### BRIEF DESCRIPTION OF THE DRAWINGS

Other characteristics and advantages of the invention will readily appear from the following description of several of its embodiments, provided as non-limitative examples, and of the accompanying drawings.

On the drawings:
- Fig. 1 is a schematic view in perspective of an autonomous bioelectric physiological signal acquisition device according to an embodiment of the invention,
- Fig. 2 is a detailed schematic view of the device of figure 1 seen from the top, showing the control module and the batteries of the device,
- Fig. 3 is a detailed view of the control module of the device of figure 1, showing the printed circuit board and their components.

On the different Figures, the same reference signs designate like or similar elements.

### DETAILED DESCRIPTION

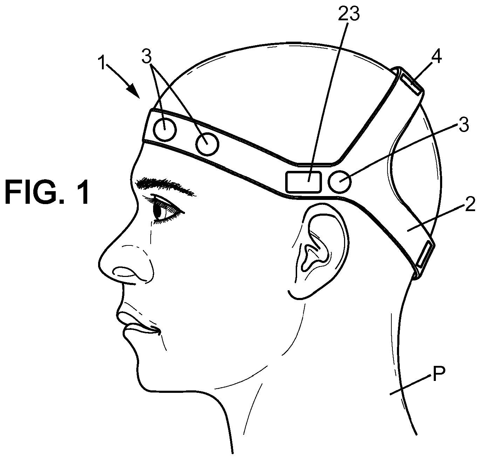

Figure 1 illustrates an autonomous bioelectric physiological signal acquisition device 1 according to an embodiment of the invention.

The device 1 is adapted to be worn by an individual, for example during a sleep period of the individual.

The device 1 is for example adapted to be worn on the head of the individual.

To this end, the device 1 can comprise one or more support members 2 adapted to at least partially surround the head of the individual so as to be held therein. In the embodiments of figure 1, the supports members 2 comprise three branches arranged so as to surround the head of the individual and maintain the device 1.

Alternatively, the device 1 may also be divided into elements, adapted to be worn on various parts of the body of the individual, for example on the head or wrist or on the chest.

The device 1 of figure 1 comprises five electrodes 3 able to be in contact with the skin of the individual and to acquire an analog signal. The analog signal can be representative of a bioelectric physiological signal.

Electrodes 3 may be reusable electrodes or disposable electrodes. Advantageously, electrodes 3 are reusable electrodes so as to simplify the daily use of the device. Electrodes 3 may be, in particular, dry electrodes. The electrodes 2 may also be of reusable or disposable textile or silicone electrodes.

In one embodiment of the invention, the device 1 comprises five electrodes 3 arranged, for example on the surface of the scalp of the person P.

The five electrodes 3 may comprise for instance two reference electrodes located on the mastoids, two measurement electrodes located on the forehead and a bias or virtual ground electrode located on the forehead. Such an arrangement is only described as a manner of example and should not be understood as being limitative with respect to the invention.

The five electrodes 3 are held by the supports members 2.

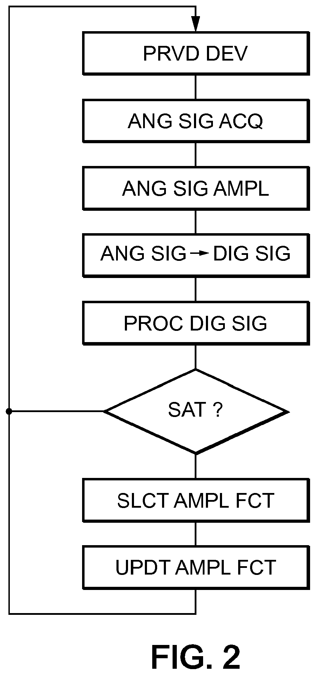

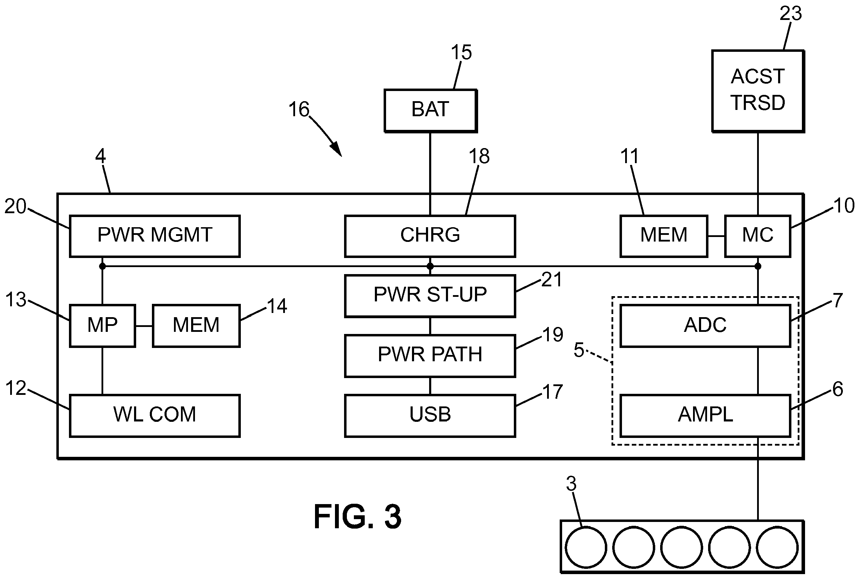

As illustrated on figures 2 and 3, the device 1 further comprises a control module 4 to process and store the signals of the electrodes.

The control module 4 comprises several acquisition chains 5, each acquisition chain 5 being associated to two electrodes 3 of the plurality of electrodes, for instance four acquisition chains 5. Each acquisition chain may advantageously receive signals from two electrodes 3, among the plurality of electrodes 5, in order to perform a differential signal measurement.

The acquisition chains 5 may be integrated in a single monolithic chip 5.

Each acquisition chain 5 comprises at least a programmable differential analog amplifier 6 and an analog digital converter 7.

The programmable differential analog amplifier 6 is able to amplify an analog signal by an amplification factor.

Each acquisition chain 5 may further comprise analog signal filters to filter the analog signal before said signal is amplified by the programmable analog amplifier 6.

The analog signal filters may comprise, for instance, low-pass and/or high-pass filters.

The input referred noise of the analog amplifiers 6 may be around 4uV at 150 Hz bandwidth. The programmable analog amplifiers 6 may thus have a high input impedance, high common mode rejection ratio, low bias current and low input referred noise to reduce the mentioned noise sources.

The analog amplifiers 6 are programmable gain amplifiers with an adjustable gain, for instance adjustable to at least a 12 times factor of amplification.

The analog digital converters 7 are able to convert an amplified analog signal into a digital signal.

The analog digital converters may have at least 4 channels and comprises 24bit delta-sigma (Δ-Σ) type ADC with an output data rate between 250SPS and 32kSPS.

The four programmable analog amplifiers 6 and the four analog digital converters 7 of the four acquisition chains 5 may form a single monolithic chip 5.

One example of thus a monolithic chip 5 is an analog front-end module with reference ADS1294 of Texas Instrument^{®}.

The control module 4 may further comprise a microcontroller 10 and a memory 11.

The microcontroller 10 is able to continuously receive the digital signal from the acquisition chains 5.

The memory 11 may be constituted of two modules: an internal memory located inside the chip of the microcontroller 10 and an external memory located outside the microcontroller 10, advantageously in proximity with the microcontroller 10. The external memory may have a larger capacity than the internal memory.

The microcontroller 10 is able to continuously store the digital signal in the memory 11.

The microcontroller 10 may be a low power microcontroller. One example of low power microcontroller is a Cortex^{®}-M0+ of ARM^{®}.

The microcontroller 10 and the memory 11 are further able to process a digital signal to determine whether said digital signal is considered saturated.

To determine whether the digital signal is considered saturated, the microcontroller 10 may be able to store a portion of the digital signal in a buffer memory of the memory 11.

Said portion of the digital signal may preferably have a duration of at least one second, for instance 5 seconds.

The microcontroller 10 may then be able to compute a ratio of saturated values to non-saturated values in the stored digital signal.

The microcontroller 10 then compares said ratio to a predefined threshold.

If said ratio is over said predefined threshold, the digital signal is considered saturated.

If said digital signal is considered saturated, the microcontroller 10 may then select, in a list of predefined amplification factors, an amplification factor lower than the amplification factor currently applied to the digital signal by the programmable analog amplifier.

The list of predefined amplification factors may be stored in the memory 11, in particular on the internal memory of the microcontroller 10.

Said selection in the list of amplification factor may, in practice, gives an index value referencing the amplification factor in the list of predefined amplification factors, for instance an index value between 1 and a max value, for instance 7.

The microcontroller 10 can then update the amplification factor of the programmable analog amplifier to the selected amplification factor.

To this aim, the microcontroller 10 may for instance transmit said index value to the programmable analog amplifier.

This way, it is possible to dynamically adjust the amplification level to the quality of the electrode skin contact and to avoid saturated and non-usable signals while still having the highest possible signal levels.

The device 1 may also comprise a microprocessor 13.

The microprocessor 13 is able to communicate with the microcontroller 10 in order to receive the digital signal stored in the memory 11.

The microprocessor 13 is also able to process said digital signal, for example to perform pattern recognition of brain states on said digital signals, for instance pattern recognition of sleep phases.

The microprocessor 13 may be able to achieve 2.5DMIPS/MHZ (with frequency at 1 GHz). The microprocessor 13 may be for instance a Cortex A9 Series Processor of ARM^{®}.

The microprocessor 13 can then store the signal in a second memory 14 of the device 1.

The second memory 14 may have a larger size than the temporary memory 11. The second memory 14 may comprise a double data rate (DDR) memory and an embedded multi-media controller (eMMC).

The device 1 may also comprise a wireless communication module 12. The microprocessor 13 may be able to control the wireless communication module 12. The wireless communication module 12 may comprises a RF transceiver chip 12 able to transmit and receive data using the Wi-Fi and the Bluetooth standards.

Moreover, the device 1 comprises at least one battery 15. The battery 15 powers the elements of the device 1, in particular at least the acquisition chains 5, the microcontroller 10 and the microprocessor 13, possibly through a power management chip 20.

The device may also comprise a power module 16 with a USB connector 17 and at least one battery charge chip 18. The battery charge chip 18 is able to receive energy from the USB connector 17 and charges said at least one battery 15.

In some embodiments, the device 1 may comprise two batteries 15 or more and the device 1 may then comprise two battery charge chips 18 respectively connected to each battery 15 and may comprise a power-path manager chip 19. The two batteries 15 may then be both connected to the power-path manager chip 19 through the respective battery charge chips 18.

The device 1 may further comprise at least one acoustic transducer 23 able to generate an acoustic stimulation to stimulate the individual.

A method for bioelectric physiological signal acquisition according to an embodiment can thus comprise the following general steps:
/a/ a step of providing an autonomous bioelectric physiological signal acquisition device,
/b/ a step of acquiring at least one analog signal as described in the present description,
/c/ a step of amplifying said analog signal as described in the present description,
/d/ a step of converting said amplified analog signal into a digital signal as described in the present description, and
/e/ a step of processing said digital signal to determine whether said digital signal is considered saturated as described in the present description.

If said digital signal is considered saturated, the following additional steps may be performed:
/f/ a step of selecting an amplification factor as described in the present description, and
/g/ a step of updating the amplification factor of the programmable analog amplifier to the selected amplification factor as described in the present description.

In some embodiment, if the digital signal is considered saturated, steps /b/ to /f/ may be reiterated until the digital signal is not considered saturated.

The amplitude of the analog signals from the electrodes is function of the quality of the skin-electrode contact. In particular, the impedance of the skin-electrode contact can vary strongly with changes in the skin-electrode contact.

Thus, if an electrode moves during the acquisition period, the contact can be deteriorated.

The deterioration of the skin-electrode contact can be slight and still allow for a measurement, in which case only the amplification factor needs to be updated, for instance lowered, to prevent saturation of the analog digital converter.

Over, in some cases the deterioration of the skin-electrode contact can be too strong and prevent further measurements of signal.

In this case, the amplification factor currently applied to the digital signal by the programmable analog amplifier may be equal to the lowest amplification factor in the list of predefined amplification factors while the digital signal is still be considered saturated.

The microcontroller 10 may then choose to power out the analog digital converter and/or the analog amplifier of the acquisition chain.

In this embodiment, at least steps /d/ to /g/ of the method according to the invention may then not be performed anymore.

This way, it is possible to take advantage of the temporary lowest quality of acquisition to save energy.

Moreover, if the digital signal is considered saturated, an amplification level change indicator may be written in the memory 11 of the device 1 by the microcontroller 10, together with an associated time stamp.

If the analog digital converter 7 of the acquisition chain 5 is powered out a channel power out indicator may be further written in said memory 11 by the microcontroller 10, together with an associated time stamp.

It may also be possible to periodically test if the quality of the electrode-skin contact has become better.

Thus, in one embodiment, if an analog digital converter and/or an analog amplifier of an acquisition chain has been powered out, the following steps may be periodically performed:
/h/ powering said analog digital converter and/or analog amplifier of an acquisition chain, and
/i/ performing at least one iteration of steps /b/ to /e/.

If the digital signal obtained from said at least one iteration is not considered saturated,
/j/ said analog digital converter and/or analog amplifier may be set back on for further iterations of steps /b/ to /e/, until said digital signal is considered saturated.

If the digital signal obtained from said at least one iteration is still considered saturated,
/k/ said analog digital converter and/or analog amplifier may then be powered out for further iterations of steps /b/ to /e/ until steps /h/ to /j/ are to be performed again.

Similarly, the level of the signal may be tested from times to times, for instance by trying a higher amplification factor for a limited time.

In one example, if the amplification factor currently applied to the digital signal by the programmable analog amplifier is not equal to the highest amplification factor in the list of predefined amplification factors, the following steps may thus be periodically performed:
/l/ selecting, in the list of predefined amplification factors, an amplification factor higher than the amplification factor currently applied to the digital signal by the programmable analog amplifier,
/m/ updating the amplification factor of the programmable analog amplifier to the selected amplification factor, and
/n/ performing at least one iteration of steps /b/ to /g/.

This way, if the digital signal is considered saturated, steps /f/ and /g/ are performed and bring back the amplification factor to its previous level. If the digital signal is not considered saturated, the increase in amplification factor will automatically be kept for further iteration of steps /b/ to /g/.

This way, it is possible to dynamically adjust the amplification level to the quality of the electrode skin contact.

The period of performing steps /l/ to /n/ and the period of performing steps /h/ to /k/ can vary and can in particular be adapted to the current power level of the battery.

In one embodiment, the microprocessor 13 can be periodically awakened from a sleep state to process the signal stored in the temporary memory 11.

Since the microprocessor 13 has significantly more power consumption compared to the acquisition chain 5 and the microcontroller 10, the microprocessor 13 may only be used in case of need or periodically.

The microprocessor 13 may thus for instance be periodically awakened from a sleep state to receive the digital signal stored in the memory 11 and process said digital signal stored in the memory 11.

The microprocessor 13 may also communicate with the microcontroller 10 to receive the amplification level change indicator(s) and/or the channel power out indicator(s) written in the memory 11.

Moreover, if the microprocessor 13 detects that the signal recorded in the temporary memory 11 is representative of a stimulation-ready state of the individual, the microprocessor 13 may then be continuously kept in an awaken state and command the acoustic transducer 23 to generate acoustic stimulations to the individual.

## Claims

1. A method for bioelectric physiological signal acquisition to dynamically adjust an amplification level to a quality of an electrode-skin contact, comprising:
/a/ providing an autonomous bioelectric physiological signal acquisition device (1) comprising at least one electrode (3) and at least one acquisition chain (5) associated to said at least one electrode,
/b/ acquiring at least one analog signal representative of a bioelectric physiological signal by means of said at least one electrode (3),
/c/ amplifying said analog signal by an amplification factor by means of a programmable analog amplifier (6) of said at least one acquisition chain (5),
/d/ converting said amplified analog signal in a digital signal by means of an analog digital converter (7) of said at least one acquisition chain (5),
/e/ processing said digital signal to determine whether said digital signal is considered saturated,
if said digital signal is considered saturated,
/f/ selecting, in a list of predefined amplification factors, an amplification factor lower than the amplification factor currently applied to the digital signal by the programmable analog amplifier (6), and
/g/ updating the amplification factor of the programmable analog amplifier (6) to the selected amplification factor,
wherein if the amplification factor currently applied to the digital signal by the programmable analog amplifier (6) is equal to the lowest amplification factor in the list of predefined amplification factors and the digital signal is considered saturated, the analog digital converter (7) and/or the programmable analog amplifier (6) of the acquisition chain (5) are powered out and steps /d/ to /g/ are not performed anymore.

2. The method of claim 1, wherein, steps /b/ to /g/ are reiterated continuously during a period of acquisition of the device (1).

3. The method of claim 1, wherein if an analog digital converter (7) and/or a programmable analog amplifier (6) of an acquisition chain (5) has been powered out, the following steps are periodically performed:
/h/ powering said analog digital converter (7) and/or programmable analog amplifier (6),
/i/ performing at least one iteration of steps /b/ to /e/,
if the digital signal obtained from said at least one iteration is not considered saturated,
/j/ setting back on said analog digital converter (7) and/or programmable analog amplifier (6) for further iterations of steps /b/ to /e/ until said digital signal is considered saturated, or if the digital signal obtained from said at least one iteration is considered saturated,
/k/ powering out said analog digital converter (7) and/or programmable analog amplifier (6) for further iterations of steps /b/ to /g/ until step /h/ is performed again.

4. The method of anyone of claim 1 to 3, wherein, if the amplification factor currently applied to the digital signal by the programmable analog amplifier (6) is not equal to the highest amplification factor in the list of predefined amplification factors, the following steps are periodically performed:
/l/ selecting, in the list of predefined amplification factors, an amplification factor higher than the amplification factor currently applied to the digital signal by the programmable analog amplifier (6),
/m/ updating the amplification factor of the programmable analog amplifier (6) to the selected amplification factor, and
/n/ performing at least one iteration of steps /b/ to /g/.

5. The method of anyone of claims 1 to 4, wherein to determine whether a digital signal is considered saturated,
- a portion of the digital signal is stored in a buffer memory (11), preferably a portion with a duration of at least one second,
- a ratio of saturated values to non-saturated values in the stored digital signal is computed,
- said ratio is compared to a predefined threshold, and
- if said ratio is over said predefined threshold, the digital signal is considered saturated.

6. The method of anyone of claims 1 to 5, wherein if the digital signal is considered saturated an amplification level change indicator is written in a memory (11) of the device (1) together with an associated time stamp, and preferably, if the analog digital converter (7) of the acquisition chain (5) is powered out a channel power out indicator is further written in said memory (11) together with an associated time stamp.

7. The method of anyone of claims 1 to 6, wherein the provided autonomous bioelectric physiological signal acquisition device (1) comprises a plurality of electrodes (3) and a plurality of respective acquisition chains (5) respectively associated to said electrodes (3),
wherein said plurality of electrodes (3) acquires a plurality of respective analog signals representative of a plurality of respective bioelectric physiological signals,
wherein said plurality of analog signals are amplified by a plurality of amplification factor by means of a respective plurality of programmable analog amplifier (6) of said plurality of acquisition chains (5),
wherein said plurality of amplified analog signals are converted in a respective plurality of digital signals by means of a respective plurality of analog digital converters (7) of said plurality of acquisition chains (5),
wherein said digital signals are independently processed to determine whether each digital signal of the plurality of digital signals is considered saturated,
and wherein for each digital signal of the plurality of digital signals that is considered saturated,
an amplification factor, lower than the amplification factor currently applied to said digital signal by the programmable analog amplifier (6) associated to said digital signal, is selected in a list of predefined amplification factors, and
the amplification factor of the programmable analog amplifier (6) associated to said digital signal is updated to the selected amplification factor.

8. An autonomous bioelectric physiological signal acquisition device comprising
- at least one electrode (3) able to be in contact with the skin of an individual and to acquire an analog signal representative of a bioelectric physiological signal,
- at least one acquisition chain (5) associated to said at least one electrode (3) and comprising:
• a programmable analog amplifier (6) able to amplify an analog signal by an amplification factor, and
• an analog digital converter (7) able to convert an amplified analog signal in a digital signal,
- a microcontroller (10) and a memory (11) able to
• process a digital signal to determine whether said digital signal is considered saturated, and
• if said digital signal is considered saturated, select, in a list of predefined amplification factors, an amplification factor lower than the amplification factor currently applied to the digital signal by the programmable analog amplifier (6),
update the amplification factor of the programmable analog amplifier (6) to the selected amplification factor, and
• if the amplification factor currently applied to the digital signal by the programmable analog amplifier is equal to the lowest amplification factor in the list of predefined amplification factors and the digital signal is considered saturated, power out the analog digital converter (7) and/or the programmable analog amplifier (6) of the acquisition chain.

9. The device according to claim 8, the device comprising a plurality of electrodes (3) and a plurality of respective acquisition chains (5) respectively associated to said electrodes (3).

10. Device according to claim 8 or 9, wherein the acquisition chains (5) are integrated in a single monolithic chip incorporating programmable analog amplifiers and analog digital converters.

11. Device according to anyone of claims 8 to 10, wherein the analog digital converters (7) are of the 24bit delta-sigma (Δ-Σ) type with an output data rate between 250SPS and 32kSPS.

12. Device according to anyone of claims 8 to 11, wherein the device (1) comprise at least one battery (15) powering at least the at least one acquisition chain (5), the microcontroller (10) and the memory (11).

13. The device according to anyone of claims 8 to 12, further comprising a microprocessor (13) able to receive the digital signal from the microcontroller (10) and to process said digital signal.

## Patentansprüche

1. Verfahren zur bioelektrischen physiologischen Signalerfassung zum dynamischen Anpassen eines Verstärkungsgrades an eine Qualität eines Kontakts zwischen Elektrode und Haut, umfassend:
/a/ Bereitstellen einer autonomen bioelektrischen physiologischen Signalerfassungsvorrichtung (1), mindestens eine Elektrode (3) und mindestens eine Erfassungskette (5) umfassend, die der mindestens einen Elektrode zugeordnet ist,
/b/ Erfassen mindestens eines analogen Signals, das repräsentativ für ein bioelektrisches physiologisches Signal ist, anhand der mindestens einen Elektrode (3),
/c/ Verstärken des analogen Signals um einen Verstärkungsfaktor anhand eines programmierbaren analogen Verstärkers (6) der mindestens einen Erfassungskette (5),
/d/ Umwandeln des verstärkten analogen Signals in ein digitales Signal anhand eines Analog-Digital-Wandlers (7) der mindestens einen Erfassungskette (5),
/e/ Verarbeiten des digitalen Signals zum Bestimmen, ob das digitale Signal als gesättigt angesehen wird,
falls das digitale Signal als gesättigt angesehen wird,
/f/ Auswählen aus einer Liste von vordefinierten Verstärkungsfaktoren eines Verstärkungsfaktors der geringer ist, als der derzeit durch den programmierbaren analogen Verstärker (6) auf das digitale Signal angewandte Verstärkungsfaktor, und
/g/ Aktualisieren des Verstärkungsfaktors des programmierbaren analogen Verstärkers (6) auf den ausgewählten Verstärkungsfaktor,
wobei, falls der derzeit durch den programmierbaren analogen Verstärker (6) auf das digitale Signal angewandte Verstärkungsfaktor gleich dem niedrigsten Verstärkungsfaktor in der Liste von vordefinierten Verstärkungsfaktoren ist, und das digitale Signal als gesättigt angesehen wird, der Analog-Digital-Wandler (7) und/oder programmierbare analoge Verstärker (6) der Erfassungskette (5) ausgeschaltet werden und die Schritte /d/ bis /g/ nicht mehr ausgeführt werden.

2. Verfahren nach Anspruch 1, wobei die Schritte /b/ bis /g/ während eines Zeitraums einer Erfassung der Vorrichtung (1) kontinuierlich wiederholt werden.

3. Verfahren nach Anspruch 1, wobei, falls ein Analog-Digital-Wandler (7) und/oder programmierbarer analoger Verstärker (6) einer Erfassungskette (5) ausgeschaltet worden sind, die folgenden Schritte periodisch ausgeführt werden:
/h/ Einschalten des Analog-Digital-Wandlers (7) und/oder programmierbaren analogen Verstärkers (6),
/i/ Ausführen mindestens einer Wiederholung der Schritte /b/ bis /e/,
falls das aus der mindestens einen Wiederholung erhaltene digitale Signal nicht als gesättigt betrachtet wird,
/j/ Zurücksetzen am Analog-Digital-Wandler (7) und/oder programmierbaren analogen Verstärker (6) für weitere Wiederholungen der Schritte /b/ bis /e/ bis das digitale Signal als gesättigt betrachtet wird,
falls das aus der mindestens einen Wiederholung erhaltene digitale Signal als gesättigt betrachtet wird,
/k/ Ausschalten des Analog-Digital-Wandlers (7) und/oder programmierbaren analogen Verstärkers (6) für weitere Wiederholungen der Schritte /b/ bis /g/, bis Schritt /h/ erneut ausgeführt wird.

4. Verfahren nach einem der Ansprüche 1 bis 3, wobei, falls der derzeit durch den programmierbaren analogen Verstärker (6) auf das digitale Signal angewandte Verstärkungsfaktor nicht gleich dem höchsten Verstärkungsfaktor in der Liste von vordefinierten Verstärkungsfaktoren ist, die folgenden Schritte periodisch ausgeführt werden:
/l/ Auswählen aus der Liste von vordefinierten Verstärkungsfaktoren eines höheren Verstärkungsfaktors als den derzeit durch den programmierbaren analogen Verstärker (6) auf das digitale Signal angewandten Verstärkungsfaktor,
/m/ Aktualisieren des Verstärkungsfaktors des programmierbaren analogen Verstärkers (6) auf den ausgewählten Verstärkungsfaktor, und
/n/ Ausführen mindestens einer Wiederholung der Schritte /b/ bis /g/.

5. Verfahren nach einem der Ansprüche 1 bis 4, wobei zum Bestimmen, ob ein digitales Signal als gesättigt angesehen wird,
- ein Abschnitt des digitalen Signals in einem Pufferspeicher (11), vorzugsweise ein Abschnitt mit einer Dauer von mindestens einer Sekunde, gespeichert wird,
- ein Verhältnis von gesättigten Werten zu nicht gesättigten Werten in dem gespeicherten digitalen Signal berechnet wird,
- das Verhältnis mit einem vordefinierten Schwellenwert verglichen wird, und
- falls das Verhältnis über dem vordefinierten Schwellenwert liegt, das digitale Signal als gesättigt angesehen wird.

6. Verfahren nach einem der Ansprüche 1 bis 5, wobei, falls das digitale Signal als gesättigt angesehen wird, ein Änderungsindikator des Verstärkungsgrades gemeinsam mit einem zugeordneten Zeitstempel in einen Speicher (11) der Vorrichtung (1) geschrieben wird, und falls der Analog-Digital-Wandler (7) der Erfassungskette (5) vorzugsweise ausgeschaltet wird, ein Ausschaltindikator des Kanals weiter gemeinsam mit einem zugeordneten Zeitstempel in den Speicher (11) geschrieben wird.

7. Verfahren nach einem der Ansprüche 1 bis 6, wobei die bereitgestellte autonome bioelektrische physiologische Signalerfassungsvorrichtung (1) eine Vielzahl von Elektroden (3) und eine Vielzahl von jeweiligen Erfassungsketten (5) umfasst, die jeweils den Elektroden (3) zugeordnet sind,
wobei die Vielzahl von Elektroden (3) eine Vielzahl von jeweiligen analogen Signalen erfasst, die repräsentativ für eine Vielzahl von jeweiligen bioelektrischen physiologischen Signalen sind,
wobei die Vielzahl von analogen Signalen durch eine Vielzahl von Verstärkungsfaktoren anhand einer jeweiligen Vielzahl von programmierbaren analogen Verstärkern (6) der Vielzahl von Erfassungsketten (5) verstärkt wird, wobei die Vielzahl von verstärkten analogen Signalen anhand einer jeweiligen Vielzahl von Analog-Digital-Wandlern (7) der Vielzahl von Erfassungsketten (5) in eine jeweilige Vielzahl von digitalen Signalen umgewandelt wird,
wobei die digitalen Signale unabhängig verarbeitet werden, um zu bestimmen, ob jedes digitale Signal der Vielzahl von digitalen Signalen als gesättigt angesehen wird, und wobei für jedes digitale Signal der Vielzahl von digitalen Signalen, das als gesättigt angesehen wird,
ein Verstärkungsfaktor, der geringer ist, als der derzeit durch den programmierbaren analogen Verstärker (6), der dem digitalen Signal zugeordnet ist, auf das digitale Signal angewandte Verstärkungsfaktor, aus einer Liste von vordefinierten Verstärkungsfaktoren ausgewählt wird, und
der Verstärkungsfaktor des programmierbaren analogen Verstärkers (6), der dem digitalen Signal zugeordnet ist, auf den ausgewählten Verstärkungsfaktor aktualisiert wird.

8. Autonome bioelektrische physiologische Signalerfassungsvorrichtung, umfassend
- mindestens eine Elektrode (3), die imstande ist, in Kontakt mit der Haut einer Person zu sein und ein analoges Signal zu erfassen, das repräsentativ für ein bioelektrisches physiologisches Signal ist,
- mindestens eine Erfassungskette (5), die der mindestens einen Elektrode (3) zugeordnet ist, und die Folgendes umfasst:
• einen programmierbaren analogen Verstärker (6), der imstande ist, ein analoges Signal um einen Verstärkungsfaktor zu verstärken, und
• einen Analog-Digital-Wandler (7), der imstande ist, ein verstärktes analoges Signal in ein digitales Signal umzuwandeln,
- einen Mikrocontroller (10) und einen Speicher (11), die imstande sind zum
• Verarbeiten eines digitalen Signals, um zu bestimmen, ob das digitale Signal als gesättigt angesehen wird, und
• falls das digitale Signals als gesättigt angesehen wird,
Auswählen aus einer Liste von vordefinierten Verstärkungsfaktoren eines Verstärkungsfaktors der geringer ist, als der derzeit durch den programmierbaren analogen Verstärker (6) auf das digitale Signal angewandte Verstärkungsfaktor, Aktualisieren des Verstärkungsfaktors des programmierbaren analogen Verstärkers (6) auf den ausgewählten Verstärkungsfaktor, und
• falls der derzeit durch den programmierbaren analogen Verstärker auf das digitale Signal angewandte Verstärkungsfaktor gleich dem niedrigsten Verstärkungsfaktor in der Liste von vordefinierten Verstärkungsfaktoren ist, und das digitale Signal als gesättigt angesehen wird, der Analog-Digital-Wandler (7) und/oder programmierbare analoge Verstärker (6) der Erfassungskette ausgeschaltet werden.

9. Vorrichtung nach Anspruch 8, wobei die Vorrichtung eine Vielzahl von Elektroden (3) und eine Vielzahl von jeweiligen Erfassungsketten (5) umfasst, die jeweils den Elektroden (3) zugeordnet sind.

10. Vorrichtung nach Anspruch 8 oder 9, wobei die Erfassungsketten (5) in einen einzelnen monolithischen Chip integriert sind, der programmierbare analoge Verstärker und Analog-Digital-Wandler einbezieht.

11. Vorrichtung nach einem der Ansprüche 8 bis 10, wobei die Analog-Digital-Wandler (7) vom 24-Bit Delta-Sigma (Δ - Σ) Typ mit einer Ausgaberate zwischen 250SPS und 32kSPS sind.

12. Vorrichtung nach einem der Ansprüche 8 bis 11, wobei die Vorrichtung (1) mindestens eine Batterie (15) umfasst, die mindestens die mindestens eine Erfassungskette (5), den Mikrocontroller (10) und den Speicher (11) mit Strom versorgt.

13. Vorrichtung nach einem der Ansprüche 8 bis 12, die weiter einen Mikroprozessor (13) umfasst, der imstande ist, das digitale Signal von dem Mikrocontroller (10) zu empfangen und das digitale Signal zu verarbeiten.

## Revendications

1. Procédé pour l'acquisition de signaux physiologiques bioélectriques pour ajuster de manière dynamique un niveau d'amplification à une qualité d'un contact électrode-peau, comprenant :
/a/ la fourniture d'un dispositif autonome d'acquisition de signaux physiologiques bioélectriques (1) comprenant au moins une électrode (3) et au moins une chaîne d'acquisition (5) associée à ladite au moins une électrode,
/b/ l'acquisition d'au moins un signal analogique représentatif d'un signal physiologique bioélectrique au moyen de ladite au moins une électrode (3),
/c/ l'amplification dudit signal analogique par un facteur d'amplification au moyen d'un amplificateur analogique programmable (6) de ladite au moins une chaîne d'acquisition (5),
/d/ la conversion dudit signal analogique amplifié en un signal numérique au moyen d'un convertisseur analogique-numérique (7) de ladite au moins une chaîne d'acquisition (5),
/e/ le traitement dudit signal numérique pour déterminer si ledit signal numérique est considéré comme saturé,
si ledit signal numérique est considéré comme saturé,
/f/ la sélection, dans une liste de facteurs d'amplification prédéfinis, d'un facteur d'amplification plus faible que le facteur d'amplification actuellement appliqué au signal numérique par l'amplificateur analogique programmable (6), et
/g/ la mise à jour du facteur d'amplification de l'amplificateur analogique programmable (6) vers le facteur d'amplification sélectionné,
dans lequel si le facteur d'amplification actuellement appliqué au signal numérique par l'amplificateur analogique programmable (6) est égal au facteur d'amplification le plus faible dans la liste de facteurs d'amplification prédéfinis et le signal numérique est considéré comme saturé, le convertisseur analogique-numérique (7) et/ou l'amplificateur analogique programmable (6) de la chaîne d'acquisition (5) sont mis hors tension et les étapes /d/ à /g/ ne sont plus réalisées.

2. Procédé selon la revendication 1, dans lequel, les étapes /b/ à /g/ sont réitérées en continu pendant une période d'acquisition du dispositif (1).

3. Procédé selon la revendication 1, dans lequel si un convertisseur analogique-numérique (7) et/ou un amplificateur analogique programmable (6) d'une chaîne d'acquisition (5) a été mis hors tension, les étapes suivantes sont réalisées de manière périodique :
/h/ la mise sous tension dudit convertisseur analogique-numérique (7) et/ou dudit amplificateur analogique programmable (6),
/i/ la réalisation d'au moins une itération des étapes /b/ à /e/,
si le signal numérique obtenu à partir de ladite au moins une itération n'est pas considéré comme saturé,
/j/ la remise sous tension dudit convertisseur analogique-numérique (7) et/ou dudit amplificateur analogique programmable (6) pour des itérations ultérieures des étapes /b/ à /e/ jusqu'à ce que ledit signal numérique soit considéré comme saturé,
ou si le signal numérique obtenu à partir de ladite au moins une itération est considéré comme saturé,
/k/ la mise hors tension dudit convertisseur analogique-numérique (7) et/ou dudit amplificateur analogique programmable (6) pour des itérations ultérieures des étapes /b/ à /g/ jusqu'à ce que l'étape /h/ soit réalisée à nouveau.

4. Procédé selon l'une quelconque des revendications 1 à 3, dans lequel, si le facteur d'amplification actuellement appliqué au signal numérique par l'amplificateur analogique programmable (6) n'est pas égal au facteur d'amplification le plus élevé dans la liste de facteurs d'amplification prédéfinis, les étapes suivantes sont réalisées de manière périodique :
/l/ la sélection, dans la liste de facteurs d'amplification prédéfinis, d'un facteur d'amplification plus élevé que le facteur d'amplification actuellement appliqué au signal numérique par l'amplificateur analogique programmable (6),
/m/ la mise à jour du facteur d'amplification de l'amplificateur analogique programmable (6) vers le facteur d'amplification sélectionné, et
/n/ la réalisation d'au moins une itération des étapes /b/ à /g/.

5. Procédé selon l'une quelconque des revendications 1 à 4, dans lequel pour déterminer si un signal numérique est considéré comme saturé,
- une portion du signal numérique est stockée dans une mémoire tampon (11), de préférence une portion ayant une durée d'au moins une seconde,
- un rapport de valeurs saturées sur des valeurs non saturées dans le signal numérique stocké est calculé,
- ledit rapport est comparé avec un seuil prédéfini, et
- si ledit rapport est au-dessus dudit seuil prédéfini, le signal numérique est considéré comme saturé.

6. Procédé selon l'une quelconque des revendications 1 à 5, dans lequel si le signal numérique est considéré comme saturé, un indicateur de changement de niveau d'amplification est écrit dans une mémoire (11) du dispositif (1) conjointement avec un horodatage associé, et de préférence, si le convertisseur analogique-numérique (7) de la chaîne d'acquisition (5) est mis hors tension, un indicateur de mise hors tension de canal est en outre écrit dans ladite mémoire (11) conjointement avec un horodatage associé.

7. Procédé selon l'une quelconque des revendications 1 à 6, dans lequel le dispositif autonome d'acquisition de signaux physiologiques bioélectriques (1) prévu comprend une pluralité d'électrodes (3) et une pluralité de chaînes d'acquisition (5) respectives respectivement associées auxdites électrodes (3),
dans lequel ladite pluralité d'électrodes (3) acquiert une pluralité de signaux analogiques respectifs représentatifs d'une pluralité de signaux physiologiques bioélectriques respectifs,
dans lequel ladite pluralité de signaux analogiques sont amplifiés par une pluralité de facteurs d'amplification au moyen d'une pluralité respective d'amplificateurs analogiques programmables (6) de ladite pluralité de chaînes d'acquisition (5),
dans lequel ladite pluralité de signaux analogiques amplifiés sont convertis en une pluralité respective de signaux numériques au moyen d'une pluralité respective de convertisseurs analogique-numérique (7) de ladite pluralité de chaînes d'acquisition (5),
dans lequel lesdits signaux numériques sont indépendamment traités pour déterminer si chaque signal numérique de la pluralité de signaux numériques est considéré comme saturé,
et dans lequel pour chaque signal numérique de la pluralité de signaux numériques qui est considéré comme saturé,
un facteur d'amplification, plus faible que le facteur d'amplification actuellement appliqué audit signal numérique par l'amplificateur analogique programmable (6) associé audit signal numérique, est sélectionné dans une liste de facteurs d'amplification prédéfinis, et
le facteur d'amplification de l'amplificateur analogique programmable (6) associé audit signal numérique est mis à jour vers le facteur d'amplification sélectionné.

8. Dispositif autonome d'acquisition de signaux physiologiques bioélectriques comprenant
- au moins une électrode (3) apte à être en contact avec la peau d'un individu et à acquérir un signal analogique représentatif d'un signal physiologique bioélectrique,
- au moins une chaîne d'acquisition (5) associée à ladite au moins une électrode (3) et comprenant :
• un amplificateur analogique programmable (6) apte à amplifier un signal analogique par un facteur d'amplification, et
• un convertisseur analogique-numérique (7) apte à convertir un signal analogique amplifié en un signal numérique,
- un microcontrôleur (10) et une mémoire (11) aptes à
• traiter un signal numérique pour déterminer si ledit signal numérique est considéré comme saturé, et
• si ledit signal numérique est considéré comme saturé,
sélectionner, dans une liste de facteurs d'amplification prédéfinis, un facteur d'amplification plus faible que le facteur d'amplification actuellement appliqué au signal numérique par l'amplificateur analogique programmable (6),
mettre à jour le facteur d'amplification de l'amplificateur analogique programmable (6) vers le facteur d'amplification sélectionné, et
• si le facteur d'amplification actuellement appliqué au signal numérique par l'amplificateur analogique programmable est égal au facteur d'amplification le plus faible dans la liste de facteurs d'amplification prédéfinis et le signal numérique est considéré comme saturé, mettre hors tension le convertisseur analogique-numérique (7) et/ou l'amplificateur analogique programmable (6) de la chaîne d'acquisition.

9. Dispositif selon la revendication 8, le dispositif comprenant une pluralité d'électrodes (3) et une pluralité de chaînes d'acquisition (5) respectives respectivement associées auxdites électrodes (3).

10. Dispositif selon la revendication 8 ou 9, dans lequel les chaînes d'acquisition (5) sont intégrées dans une unique puce monolithique incorporant des amplificateurs analogiques programmables et des convertisseurs analogique-numérique.

11. Dispositif selon l'une quelconque des revendications 8 à 10, dans lequel les convertisseurs analogique-numérique (7) sont du type 24 bits delta-sigma (Δ-Σ) avec un débit de données de sortie entre 250 SPS et 32 kSPS.

12. Dispositif selon l'une quelconque des revendications 8 à 11, dans lequel le dispositif (1) comprend au moins une batterie (15) alimentant au moins l'au moins une chaîne d'acquisition (5), le microcontrôleur (10) et la mémoire (11).

13. Dispositif selon l'une quelconque des revendications 8 à 12, comprenant en outre un microprocesseur (13) apte à recevoir le signal numérique provenant du microcontrôleur (10) et à traiter ledit signal numérique.
